# FASCICULE DE BREVET EUROPEEN

(11) **EP 1 279 381 B1**
(45) Date de publication et mention de la délivrance du brevet: **27.06.2007**
(21) Numéro de dépôt: 02291143.2
(22) Date de dépôt: 06.05.2002
(51) Int. Cl.: A61F 2/00, A61F 13/20, A61F 13/26

(54) **Dispositif intra-vaginal de prévention de l'incontinence urinaire féminine**
Intravaginalvorrichtung zur Verhinderung von weiblicher Harninkontinenz
Intravaginal device for prevention of femal urinary incontinence

(30) Priorité: 07.05.2001 FR 0106063
(43) Date de publication de la demande: 29.01.2003
(73) Titulaire: Guerquin, Bernard, 57800 Cocheren (FR)
(72) Inventeur: Guerquin, Bernard, 57800 Cocheren (FR)
(74) Mandataire: Lerner, François

(56) Documents cités:
- EP-A- 0 933 069
- WO-A-00/78261
- WO-A-95/05790
- FR-A- 2 297 031
- US-A- 3 683 915
- US-A- 5 785 640
- US-A- 6 142 928

## Description

L'invention concerne un dispositif de prévention de l'incontinence urinaire féminine à l'effort destiné à être disposé de manière longitudinale dans le vagin.

Les dispositifs proposés jusqu'alors prévoient de relever la jonction urétro-vésicale par une prothèse disposée dans le vagin en regard de cette jonction.

Le document US-5 785 640 décrit un dispositif de prévention de l'incontinence féminine à l'effort dans lequel la partie la plus antérieure est formée de deux Saillies destinées à appliquer une pression localisée au col vésical.

Le document WO 95 05790 décrit un dispositif de prévention de l'incontinence féminine à l'effort dans lequel deux parties sont écartées élastiquement l'une de l'autre afin qu'elles exercent des pressions localisées à des parties opposées des parois du vagin. Ces parties opposées des parois se trouvent au niveau du col vésical, et aucune partie du dispositif ne s'étend plus en avant dans la zone sous-urétrale du vagin.

Ces prothèses ne sont pas toujours très efficaces, voire dans certains cas sont complètement inefficaces. Les prothèses de l'art antérieur entraînent aussi parfois des difficultés de mise en place par l'utilisatrice elle-même et peuvent requérir un suivi médical, au moins dans la période d'adaptation à une prothèse qui convient en termes de confort et de résultat aux attentes de l'utilisatrice.

La présente invention se propose de pallier ces inconvénients en proposant un dispositif prothétique simple, pouvant être fabriqué selon un procédé facile à mettre en oeuvre, que l'utilisatrice pourra mettre en place de manière correcte seule, et utiliser en particulier sur de courtes périodes, par exemple pendant ses activités sportives.

Cet objectif est atteint selon la présente invention par un dispositif qui comporte une partie proximale s'étendant entre une première extrémité libre et une deuxième extrémité, réalisée de manière à être sensiblement indéformable sous pression, et destinée à s'étendre dans la zone sous-urétrale du vagin, et une partie distale prolongeant la deuxième extrémité de la partie proximale, réalisée dans un matériau déformable sous pression de manière réversible, destinée à être placée dans la zone sous-vésicale du vagin et présentant une hauteur au plus égale à 80% de la hauteur de ladite partie proximale.

De manière préférentielle, ladite partie distale présente une hauteur comprise entre 40 et 60% de la hauteur de ladite partie proximale, et avantageusement de l'ordre de 50% de la hauteur de ladite partie proximale.

Grâce à cette configuration particulière en deux parties, on prend en compte les différences morphologiques et fonctionnelles des zones avant et arrière, respectivement sous-urétrale et sous-vésicale, de la paroi vaginale antérieure comme il sera expliqué plus loin de manière plus détaillée.

En effet, on constate dans l'incontinence urinaire d'effort féminine le rôle majeur de l'affaiblissement de la résistance de la paroi antérieure du vagin dans la zone située sous l'urètre, l'affaissement de la paroi antérieure du vagin dans la zone située sous la vessie ayant lui un effet plutôt protecteur qui doit être respecté.

La hauteur plus faible de la partie distale est importante car elle permet le respect de la déformation de la paroi vaginale dans sa partie sous vésicale, tandis que la hauteur plus importante de la partie proximale améliore la résistance mécanique de la paroi antérieure du vagin dans sa partie sous-urétrale.

De préférence, ladite partie distale présente une largeur au moins égale à 1,5 fois la largeur de ladite partie proximale.

Cette configuration élargie en partie distale tenant compte de la plus grande largeur du vagin dans sa profondeur, elle assure le bon positionnement du dispositif dans le vagin quelle que soit l'orientation initiale du dispositif lors de son introduction.

De préférence, la partie distale présente une largeur supérieure à sa hauteur. Ceci permet de prendre en compte le diamètre plus important du vagin dans sa zone sous-vésicale (arrière) par rapport à la zone sous-urétrale (avant), sans recourir à l'utilisation d'une partie distale trop encombrante et donc difficile à faire passer dans la zone avant du vagin.

Selon le ou les matériaux utilisés pour la fabrication de ce dispositif, qui seront biocompatibles au moins dans leur partie externe, le dispositif peut également remplir la fonction classique de tampon intra-vaginal périodique ou hygiénique introduit dans le vagin pendant la menstruation.

Afin de concilier les nécessités fonctionnelles avec les dimensions anatomiques féminines, la partie proximale présente, de préférence, une longueur comprise entre 1,5 et 2,5 cm, avantageusement de l'ordre de 2 cm, et la partie distale présente, de préférence, une longueur comprise entre 2 et 5 cm, avantageusement de l'ordre de 3 cm.

De manière préférentielle mais non limitative, la partie proximale présente une forme cylindrique de section circulaire ou une forme tronconique allant en s'évasant depuis la première extrémité vers la deuxième extrémité.

Selon une variante de réalisation possible, la partie proximale présente un noyau réalisé dans un matériau dur et entouré d'une enveloppe souple réalisée dans un matériau biocompatible. Dans ce cas, ledit noyau présente préférentiellement, en direction longitudinale, une forme allongée, soit cylindrique d'axe longitudinale et de section circulaire, soit oblongue.

Selon une autre variante de réalisation préférentielle, la partie distale est formée de deux boudins similaires disposés côte à côte sur leur longueur, de manière accolée ou séparée.

Ainsi, on réalise une partie distale plus large que haute adaptée à la forme de la zone sous-vésicale du vagin et qui évite également l'expulsion spontanée de l'élément de traitement hors du vagin car la partie distale est plus large que la largeur au repos de la zone sous-urétrale du vagin.

Selon un exemple de réalisation, la partie distale est réalisée en mousse, telle qu'une mousse en polyuréthane ou en tout autre matériau présentant des caractéristiques similaires de souplesse, d'élasticité et de compatibilité biologique.

Selon une forme de réalisation préférée, le dispositif comporte en outre des moyens de retrait du dispositif montés sur la première extrémité de la partie proximale, par exemple une cordelette sur laquelle il suffit de tirer pour extraire le dispositif hors du vagin.

Selon une autre forme de réalisation préférée, le dispositif comporte en outre des moyens d'insertion facilitant la mise en place du dispositif dans le vagin. Lesdits moyens d'insertion présentant avantageusement la forme d'un applicateur qui comprend un tube creux apte à contenir les parties proximale et distale et un poussoir apte à coulisser de manière télescopique dans ledit tube afin d'entraîner les parties proximale et distale dans le vagin, respectivement en regard des zones sous-urétrale et sous-vésicale de la paroi antérieure du vagin.

Plusieurs exemples de réalisation vont maintenant être décrits et illustrés ci-après. Il est entendu que la description et les dessins ne sont donnés qu'à titre indicatif et non limitatif.

II sera fait référence aux dessins annexés, dans lesquels :
- la figure 1 est une vue éclatée en projection longitudinale des différents éléments composant un dispositif de prévention de l'incontinence urinaire féminine à l'effort objet de la présente invention ;
- la figure 2A est une représentation en direction transversale de la figure 1 lorsque la partie distale est insérée dans le tube creux des moyens d'insertion ;
- la figure 2B est une représentation en direction transversale de la figure 1 (la partie distale est sortie du tube creux des moyens d'insertion) ;
- la figure.3 est une vue similaire à celle de la figure 1 pour une première variante de réalisation du dispositif selon l'invention ;
- la figure 4 est une vue en projection longitudinale d'une deuxième variante de réalisation du dispositif objet de la présente invention ;
- la figure 5 est une vue en projection longitudinale d'une troisième variante de réalisation du dispositif objet de la présente invention ;
- la figure 6 est une vue en projection longitudinale d'une quatrième variante de réalisation du dispositif objet de la présente invention ; et
- la figure 7 représente une coupe sagittale de l'anatomie vésicale et vaginale féminine avec le dispositif selon l'invention mis en place.

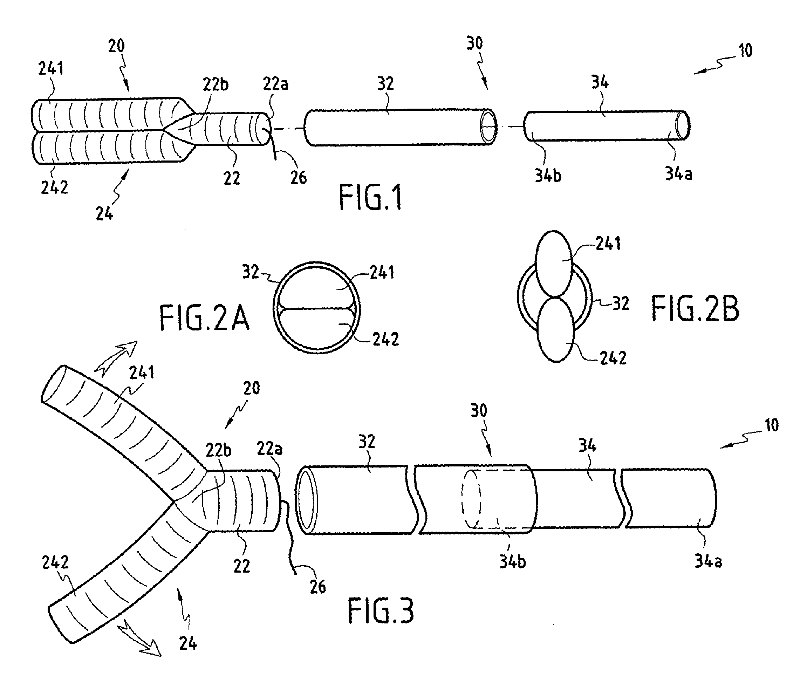

Sur la figure 1 est visible un dispositif de prévention de l'incontinence urinaire féminine à l'effort référencé 10 et comprenant trois éléments séparés, à savoir un élément de traitement 20, de forme générale longitudinale destiné à venir se loger dans le vagin, et deux éléments d'insertion formant des moyens d'insertion 30 sous forme d'un applicateur utilisé lors de la mise en place de l'élément de traitement 20.

Il est à noter que comme pour les tampons hygiéniques, l'applicateur 30 peut être omis, l'élément de traitement 20 étant alors inséré manuellement dans le vagin.

L'élément de traitement 20 se présente sous la forme générale d'un tampon intra-vaginal périodique utilisé pendant la menstruation et comporte une partie proximale 22, une partie distale 24 et, de préférence, une cordelette 26 servant de moyens de retrait du dispositif.

Les adjectifs "proximal" et "distal" se rapportent respectivement à des parties situées de manière proche et de manière éloignée de la main lors de la mise en place du dispositif dans le vagin.

Afin de repérer les différentes dimensions du dispositif 10 selon trois directions perpendiculaires entre elles, le terme « longueur » se rapporte à la plus grande dimension qui est destinée à venir se placer dans le sens de la plus grande dimension du vagin qui s'étend depuis l'entrée jusqu'au fond du vagin, le terme « hauteur » désigne la dimension destinée à venir se placer sensiblement de manière verticale dans le vagin, lorsque l'utilisatrice du dispositif est debout, et le terme « largeur » désigne la dimension destinée à venir se placer parallèlement au plan sensiblement horizontal formé par la partie arrière ou sous-vésicale du vagin qui forme une sorte de hamac.

La partie proximale 22 s'étend sur une longueur de l'ordre de 2 cm entre une première extrémité libre 22a et une deuxième extrémité 22b. La partie proximale 22 présente une forme cylindrique de révolution ayant un diamètre de l'ordre de 1,5 cm. Il est entendu que d'autres formes favorisant le retrait du dispositif hors du vagin sont possibles pour la partie proximale 22.

Cette partie proximale 22 est réalisée dans des matériaux assurant à la fois une structure sensiblement indéformable sous pression et une bio-compatibilité.

A cet effet, la partie proximale 22 est fabriquée dans un ou plusieurs matériaux lui donnant une composition essentiellement dure, c'est-à-dire qui ne se déforme pas, en particulier qui ne s'écrase pas, ou peu, sous contrainte.

La cordelette 26 destinée à l'ablation de l'élément de traitement 20 est attachée à la première extrémité 22a de la partie proximale 22 et s'étend sur une longueur de quelque centimètres au moins.

La partie distale 24 s'étend sur une longueur de l'ordre de 3 cm (d'au moins 2 cm et jusqu'à 5 cm), en prolongeant la deuxième extrémité 22b de la partie proximale 22, est constituée de deux boudins 241 et 242 identiques de forme cylindrique de révolution s'étendant en direction longitudinale.

La zone de jonction entre la partie proximale 22 et la partie distale 24 est réalisée de manière à former un changement transversal progressif de forme et de dimension.

Ces boudins 241 et 242 sont réalisés dans des matériaux assurant à la fois une structure déformable sous pression de manière réversible (avec retour élastique) et une bio-compatibilité.

Pour ce faire, la partie distale 24 est fabriquée dans un ou plusieurs matériaux donnant aux boudins 241 et 242 une composition essentiellement souple, c'est-à-dire une structure qui se déforme, en particulier qui s'écrase, tout en revenant à sa forme initiale après cessation de la contrainte exercée.

Les boudins sont par exemples réalisés dans une matière telle que celle utilisée pour les tampons hygiéniques, à savoir une matière absorbant les liquides, tissée ou non, avec des fibres naturelles (coton ou cellulose) ou synthétiques, des mousses, des éponges, des gels ou des polymères.

Bien entendu cette partie distale 24 assure tout de même une certaine résistance mécanique, faible, et qui est bien moins importante que la résistance à l'effort de la partie proximale 22.

Dans ce qui précède, la contrainte ou l'effort qui sont considérés concernent la somme de toutes les pressions exercées physiologiquement sur la paroi antérieure du vagin. S'agissant de la partie proximale 22 qui contribue à la résistance à l'effort de la partie avant (sous-urétrale) de la paroi antérieure du vagin, ces pressions comprennent notamment la pression exercée par la cavité abdominale. Pour la partie distale 24 qui contribue à la résistance à l'effort de la partie arrière (sous-vésicale) de la paroi antérieure du vagin, ces pressions comprennent notamment le poids de la vessie et sa pression interne de liquide (variables selon le degré de remplissage), ainsi que la pression exercée par la cavité abdominale.

Dans le cas de la variante de réalisation illustrée sur la figure 1, les deux boudins 241 et 242 sont accolés sur leur longueur.

L'agencement constitué par ces deux boudins donne une forme plus large que haute à la partie distale 24. Cette configuration assure surtout une forme plus large de la partie distale 24 vis à vis de la partie proximale 22, ce qui est important comme il sera expliqué plus loin.

De préférence, la partie distale 24 présente une largeur au moins égale à 1,5 fois la largeur de la partie proximale 22 et de préférence égale à 2 fois cette largeur.

En outre, on prévoit que la partie distale 24 présente une hauteur au plus égale à 80% de la hauteur de la partie proximale 22, avantageusement comprise entre 40 et 60% de la hauteur de la partie proximale 22 et de préférence sensiblement égale à 50% de la hauteur de la partie proximale 22. On utilisera par exemple une partie distale 24 ayant une largeur comprise entre 2,5 et 3,5 cm pour une hauteur maximale de l'ordre de 1,5 cm, préférentiellement autour de 1 cm.

Les deux éléments d'insertion formant les moyens d'insertion 30 (ou applicateur) sont constitués par un tube creux 32 et par un poussoir 34. Le tube 32 forme un logement de section circulaire pour l'élément de traitement 20 avant sa mise en place. Le diamètre de ce logement est sensiblement égal ou légèrement supérieur au diamètre extérieur de la partie proximale 22, elle même de l'ordre de 1 à 1,5 cm. Les deux boudins 241 et 242 ont un diamètre significativement plus petit que celui de la partie proximale 22, sans pour autant être inférieur ou égal à la moitié du diamètre de la partie proximale 22 du fait de la consistance assez souple de la partie distale 24.

Ainsi, comme on peut le voir sur la figure 2A, les deux boudins 241 et 242 sont écrasés dans le tube 32 avant la mise en place du dispositif par l'utilisatrice.

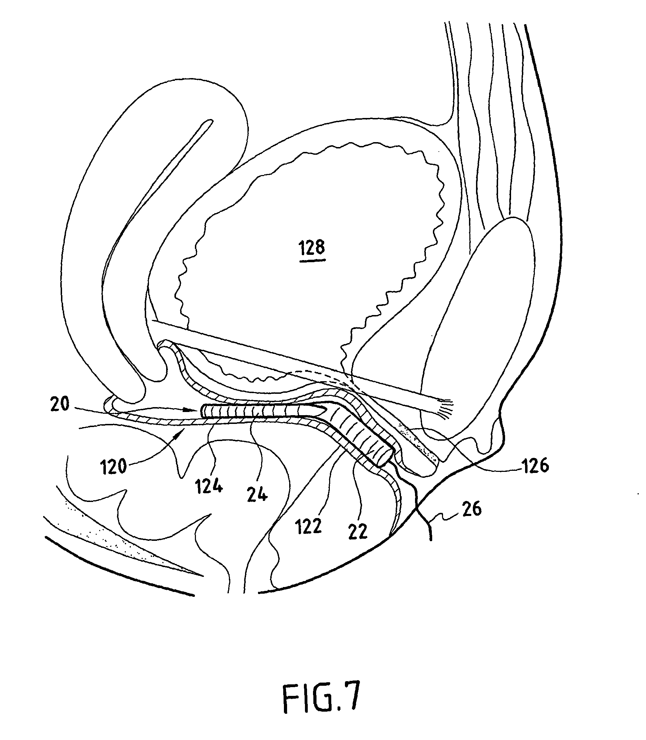

On comprend que pour cette mise en place du dispositif, l'utilisatrice appuie sur l'extrémité proximale 34a du poussoir 34 qui est entraîné et guidé en coulissement à l'intérieur du logement du tube 32 de manière télescopique. De cette manière, l'élément de traitement 20 sort progressivement de son logement et vient se placer dans le vagin de l'utilisatrice comme illustré sur la figure 7.

La première variante de réalisation représentée sur la figure 3 prévoit que les deux boudins 241 et 242 ne sont pas accolés, sur leur longueur mais séparables en s'écartant l'un de l'autre de part et d'autre de la direction longitudinale comme indiqué par les flèches à gauche de la figure 3. Cette conformation est en particulier adaptée aux femmes ménopausées qui présentent un vagin distendu par rapport au vagin des femmes jeunes, la largeur de la partie distale 24 étant plus grande avec des boudins non accolés et permettant un soutien de la zone arrière du vagin sur une plus grande étendue latérale que lorsque les boudins 241 et 242 sont accolés.

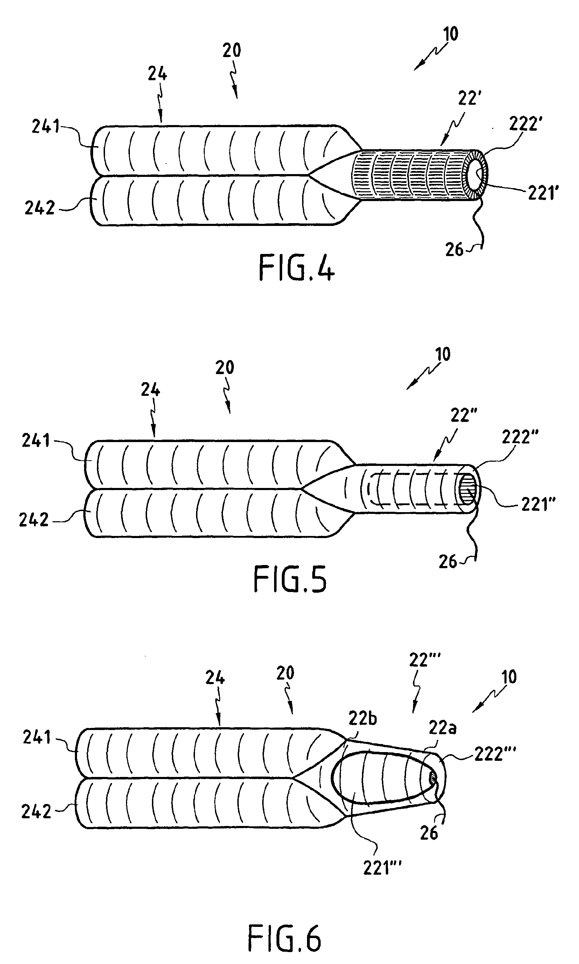

Les figures 4 à 6 illustrent des variantes de réalisation de la partie proximale 22, la partie distale 24 étant représentée sur ces figures à l'identique de celle de la figure 1 mais on peut envisager qu'elle soit identique à la partie distale 24 de la figure 3.

Dans la deuxième variante de réalisation de la figure 4, on prévoit que la partie proximale 22', cylindrique se section circulaire, comporte un noyau 221' entouré d'une enveloppe 222'. Le noyau 221' est souple est réalisé dans la même matière que la partie distale 24. L'enveloppe 222' est réalisée dans un matériau biocompatible dur, c'est-à-dire sensiblement indéformable sous pression. Le noyau 221' est réalisé dans un matériau plus souple que le noyau 222', éventuellement dans un matériau déformable sous pression de manière réversible, et de préférence dans la même matière que la partie distale 24.

Dans la troisième variante de réalisation de la figure 5, la même structure double de la partie proximale 22' de la deuxième variante de réalisation est prévue de manière inversée quant aux caractéristiques des matériaux constituant la partie proximale 22" : cette partie proximale 22" présente un noyau réalisé 221" dans un matériau dur et entouré d'une enveloppe 222" souple réalisée dans un matériau biocompatible.

Dans les deuxième et troisième variantes de réalisation plutôt qu'une forme cylindrique d'axe longitudinale telle que représentée, le noyau 221' ou 221" peut présenter une forme allongée, par exemple oblongue.

Dans la quatrième variante de réalisation illustrée sur la figure 6, la partie proximale 22"' présente une forme tronconique allant en s'évasant depuis la première extrémité 22a vers la deuxième extrémité 22b et le noyau 222"' auquel est relié la cordelette 26 présente une forme d'olive.

Si l'on se reporte maintenant à la figure 7, après mise en place de l'élément de traitement 20 dans le vagin 120, la partie proximale 22 s'étend dans la partie avant ou sous-urétrale 122 du vagin 120 (dans le prolongement vertical de l'urètre 126 dans la position debout) et la partie distale 24 s'étend dans la partie arrière ou sous-vésicale 124 du vagin 120 (dans le prolongement vertical de la vessie 128 dans la position debout).

La partie distale 24 étant comprimée par la vessie lorsque cette dernière est pleine, les deux boudins se positionnent naturellement l'un à côté de l'autre sensiblement dans un plan horizontal pour former un soutien souple au hamac que constitue la partie arrière ou sous-vésicale 124 du vagin 120.

Ainsi, on comprend que le dispositif 100 s'étend sur sensiblement toute la longueur du vagin et qu'il se positionne automatiquement dans la bonne orientation angulaire et à la bonne position en profondeur, quelle que soit l'orientation initiale du dispositif lors de sa mise en place par l'utilisatrice.

La présente invention couvre un dispositif contre l'incontinence urinaire féminine à l'effort qui comporte l'élément de traitement 20, les moyens d'insertion 30 (tube creux 32 et poussoir 34 ou tout autre moyen équivalent) pouvant être omis sans sortir du cadre de la présente invention.

## Revendications

1. Dispositif (10) de prévention de l'incontinence urinaire féminine à l'effort destiné à être disposé de manière longitudinale dans le vagin, **caractérisé en ce qu'**il comporte :
- une partie proximale (22) s'étendant entre une première extrémité libre (22a) et une deuxième extrémité (22b), réalisée de manière à être sensiblement indéformable sous pression, et s'étendant dans la zone sous-urétrale du vagin, et
- une partie distale (24) prolongeant la deuxième extrémité (22b) de la partie proximale (22), réalisée dans un matériau déformable sous pression de manière réversible, destinée à être placée dans la zone sous-vésicale du vagin et présentant une hauteur au plus égale à 80% de la hauteur de ladite partie proximale (22).

2. Dispositif (10) selon la revendication 1, **caractérisé en ce que** la partie proximale (22) présente une longueur comprise entre 1,5 et 2,5 cm.

3. Dispositif (10) selon l'une des revendications 1 et 2, **caractérisé en ce que** la partie distale (24) présente une longueur comprise entre 2 et 5 cm.

4. Dispositif (10) selon l'une quelconque des revendications 1 à 3, **caractérisé en ce que** le dispositif s'étend sur sensiblement toute la longueur du vagin.

5. Dispositif (10) selon la revendication 1, **caractérisé en ce que** ladite partie distale (24) présente une largeur au moins égale à 1,5 fois la largeur de ladite partie proximale (22).

6. Dispositif (10) selon l'une quelconque des revendications 1 à 3, **caractérisé en ce que** la partie distale (24) présente une largeur supérieure à sa hauteur.

7. Dispositif (10) selon l'une quelconque des revendications précédentes, **caractérisé en ce que** la partie proximale (22) présente une forme cylindrique de section circulaire ou une forme tronconique allant en s'évasant depuis la première extrémité (22a) vers la deuxième extrémité (22b).

8. Dispositif (10) selon l'une quelconque des revendications précédentes, **caractérisé en ce que** la partie proximale (22" ; 22") présente un noyau (221"; 221"') réalisé dans un matériau dur et entouré d'une enveloppe (222" ; 222"') souple réalisée dans un matériau biocompatible.

9. Dispositif (10) selon la revendication 8, **caractérisé en ce que** ledit noyau (221"; 22t") présente en direction longitudinale une forme allongée, soit cylindrique d'axe longitudinale et de section circulaire, soit oblongue.

10. Dispositif (10) selon l'une quelconque des revendications précédentes, **caractérisé en ce que** la partie distale (24) est formée de deux boudins (241 ;242) similaires disposés côte à côte sur leur longueur.

11. Dispositif (10) selon l'une quelconque des revendications précédentes, **caractérisé en ce que** la partie distale (24) est réalisée en mousse.

12. Dispositif (10) selon l'une quelconque des revendications précédentes, **caractérisé en ce qu'**il comporte en outre des moyens de retrait (30) du dispositif montés sur la première extrémité de la partie proximale.

13. Dispositif (10) selon la revendication 10, **caractérisé en ce qu'**il comporte en outre un applicateur (30) formé d'un tube creux (32) apte à contenir les parties proximale (22) et distale (24) et d'un poussoir (34) apte à coulisser de manière télescopique dans ledit tube (32).

## Claims

1. Device (10) for preventing female urinary stress incontinence intended to be arranged in a longitudinal manner in the vagina, **characterised in that** it comprises:
- a proximal portion (22) which extends between a first free end (22a) and a second end (22b), which is produced so as to be substantially non-deformable under pressure, and which extends in the sub-urethral region of the vagina, and
- a distal portion (24) which extends the second end (22b) of the proximal portion (22), which is produced from a material which is deformable under pressure in a reversible manner, which is intended to be positioned in the sub-vesical region of the vagina and which has a height which is at most 80% of the height of said proximal portion (22).

2. Device (10) according to claim 1, **characterised in that** the proximal portion (22) has a length of between 1.5 and 2.5 cm.

3. Device (10) according to either claim 1 or 2, **characterised in that** the distal portion (24) has a length of between 2 and 5 cm.

4. Device (10) according to any one of claims 1 to 3, **characterised in that** the device extends over substantially the entire length of the vagina.

5. Device (10) according to claim 1, **characterised in that** said distal portion (24) has a width which is at least 1.5 times the width of said proximal portion (22).

6. Device (10) according to any one of claims 1 to 3, **characterised in that** the distal portion (24) has a width which is greater than the height thereof.

7. Device (10) according to any one of the preceding claims, **characterised in that** the proximal portion (22) has a cylindrical shape of circular cross-section or a frustoconical shape which widens from the first end (22a) towards the second end (22b).

8. Device (10) according to any one of the preceding claims, **characterised in that** the proximal portion (22"; 22"') has a core (221"; 221"') which is produced from a hard material surrounded by a flexible casing (222"; 222"') which is produced from a biocompatible material.

9. Device (10) according to claim 8, **characterised in that** said core (221"; 221"') has, in the longitudinal direction, an elongate shape, either cylindrical with a longitudinal axis and circular cross-section or oblong.

10. Device (10) according to any one of the preceding claims, **characterised in that** the distal portion (24) is formed by two similar tubular members (241; 242) which are arranged side by side over the length thereof.

11. Device (10) according to any one of the preceding claims, **characterised in that** the distal portion (24) is produced from foam.

12. Device (10) according to any one of the preceding claims, **characterised in that** it further comprises withdrawal means (30) for the device which are mounted on the first end of the proximal portion.

13. Device (10) according to claim 10, **characterised in that** it further comprises an applicator (30) which is formed by a hollow tube (32) which is able to contain the proximal portion (22) and distal portion (24) and a pusher (34) which can slide in a telescopic manner in said tube (32).

## Patentansprüche

1. Vorrichtung (10) zur Verhinderung von weiblicher Harninkontinenz bei Belastung, die dazu bestimmt ist, längs in der Vagina angeordnet zu werden, **dadurch gekennzeichnet, daß** die Vorrichtung aufweist:
- einen proximalen Teil (22), der sich zwischen einem ersten freien Ende (22a) und einem zweiten Ende (22b) erstreckt, welcher im wesentlichen unter Druck formbeständig hergestellt ist und sich in dem unteren Harnröhrenbereich der Vagina erstreckt, und
- einen distalen Teil (24), welcher das zweite Ende (22b) des proximalen Teils (22) verlängert, der aus unter Druck verformbarem Material reversibel hergestellt ist und dazu bestimmt ist, in dem unteren Harnblasenbereich der Vagina angeordnet zu werden und eine Höhe von höchstens 80 % der Höhe des proximalen Teils (22) aufweist.

2. Vorrichtung (10) nach Anspruch 1, **dadurch gekennzeichnet, daß** der proximale Teil (22) eine Länge von zwischen 1,5 und 2,5 cm hat.

3. Vorrichtung (10) nach einem der Ansprüche 1 und 2, **dadurch gekennzeichnet, daß** der distale Teil (24) eine Länge von zwischen 2 und 5 cm hat.

4. Vorrichtung (10) nach einem der Ansprüche 1 bis 3, **dadurch gekennzeichnet, daß** sich die Vorrichtung im wesentlichen auf der gesamten Länge der Vagina erstreckt.

5. Vorrichtung (10) nach Anspruch 1, **dadurch gekennzeichnet, daß** der distale Teil (24) eine Breite aufweist, die mindestens gleich dem 1,5-fachen der Breite des proximalen Teils 22 beträgt.

6. Vorrichtung (10) nach einem der Ansprüche 1 bis 3, **dadurch gekennzeichnet, daß** der distale Teil (24) eine Breite aufweist, die größer als seine Höhe ist.

7. Vorrichtung (10) nach einem der vorhergehenden Ansprüche, **dadurch gekennzeichnet, daß** der proximale Teil (22) eine zylindrische Form mit kreisrundem Querschnitt oder eine Kegelstumpfform aufweist, die von dem ersten Ende (22a) zu dem zweiten Ende (22b) hin breiter wird.

8. Vorrichtung (10) nach einem der vorhergehenden Ansprüche, **dadurch gekennzeichnet, daß** der proximale Teil (22", 22"') einen Kern (221", 221"') aufweist, der aus einem harten Material hergestellt und mit einer weichen Hülle (222", 222''') umgeben ist, die aus einem biokompatiblen Material hergestellt ist.

9. Vorrichtung (10) nach Anspruch 8, **dadurch gekennzeichnet, daß** der Kern (221", 221''') in Längsrichtung eine längliche Form aufweist, entweder zylindrisch mit Längsachse und kreisrundem Querschnitt oder länglich.

10. Vorrichtung (10) nach einem der vorhergehenden Ansprüche, **dadurch gekennzeichnet, daß** der distale Teil (24) aus zwei gleichartigen Wülsten (241, 242) gebildet ist, die auf ihrer Länge Seite an Seite angeordnet sind.

11. Vorrichtung (10) nach einem der vorhergehenden Ansprüche, **dadurch gekennzeichnet, daß** der distale Teil (24) aus Schaum hergestellt ist.

12. Vorrichtung (10) nach einem der vorhergehenden Ansprüche, **dadurch gekennzeichnet, daß** sie ferner Rückziehmittel (30) für die Vorrichtung aufweist, die auf dem ersten Ende des proximalen Teils angebracht sind.

13. Vorrichtung (10) nach Anspruch 10, **dadurch gekennzeichnet, daß** sie ferner einen Applikator (30) aufweist, der aus einem hohlen Rohr (32) gebildet ist, das geeignet ist, den proximalen Teil (22) und den distalen Teil (24) und eine Stoßvorrichtung (34) zu enthalten, die geeignet ist, teleskopartig in dem Rohr (32) zu gleiten.
